# EUROPEAN PATENT SPECIFICATION

(11) **EP 4 532 894 B1**
(45) Date of publication and mention of the grant of the patent: **04.02.2026**
(21) Application number: 22735656.5
(22) Date of filing: 03.06.2022
(51) Int. Cl.: E21B 49/00, E21B 44/00

(54) **A METHOD FOR REAL-TIME ADJUSTMENT OF AT LEAST ONE DRILLING PARAMETER DURING ROCK DRILLING BY A DRILLING MACHINE**
EIN VERFAHREN ZUR ECHTZEITEINSTELLUNG VON MINDESTENS EINEM BOHRPARAMETER WÄHREND DES FELSBOHRENS DURCH EINE BOHRMASCHINE
PROCÉDÉ DE RÉGLAGE EN TEMPS RÉEL D'AU MOINS UN PARAMÈTRE DE FORAGE PENDANT LE FORAGE DE LA ROCHE PAR UNE MACHINE DE FORAGE

(43) Date of publication of application: 09.04.2025
(73) Proprietor: Epiroc Rock Drills Aktiebolag, 701 91 Örebro (SE)
(72) Inventor: PERSSON, Anders, 663 91 Hammarö (SE); MALM, Patrik, 719 94 Vintrosa (SE)
(74) Representative: Epiroc Rock Drills AB
(86) International application number: PCT/SE2022/050552
(87) International publication number: WO 2023/234819

(56) References cited:
- US-A1- 2013 082 506
- US-A1- 2019 242 203
- US-A1- 2019 309 614

## Description

### TECHNICAL FIELD

The present disclosure relates to a method for real-time adjustment of at least one drilling parameter during rock drilling by a drilling machine. The present disclosure also relates to a control system for real-time adjustment of at least one drilling parameter during rock drilling by a drilling machine, a drilling machine, a computer program and a computer readable medium.

### BACKGROUND OF THE INVENTION

Rock drilling, i.e. when drilling a borehole in rock by excavating rock, is a challenging and demanding operation. Rock drilling is often carried out by percussion drilling, where a percussion piston, which is often operated hydraulically, is used to create a shock wave with the aid of an impact force that is generated by hydraulic pressure (percussion pressure). The shock wave is transmitted to a drill bit and hence to the rock through the drill bit. Rock drilling may additionally, or alternatively, be performed by rotary drilling, e.g. by rotating the drill bit during the rock drilling. The drill bit may additionally, or alternatively, be subjected to a feed force which forces the drill bit further into the borehole during the rock drilling operation.

During the rock drilling operation, cuttings from the rock are generated. The cuttings are preferably removed from the borehole by flushing. The flushing is typically characterized by using a flushing media, such as air and/or water, which carries the cuttings out from the borehole. Accordingly, flushing media may be injected into the borehole at a certain pressure during the rock drilling to thereby remove the cuttings from the borehole.

The rock drilling is controlled by use of one or more drilling parameters. By way of example, the one or more drilling parameters may be a specific percussion frequency, a specific feed rate and/or a specific rotational speed of the drill bit. Accordingly, the one or more drilling parameters will influence the rock drilling performance, such as the quality of the generated borehole and/or how fast the borehole is made. The one or more drilling parameters will also influence the service life of rock drilling components. As such, the one or more drilling parameters influence the quality of the borehole and the cost for making the borehole. Such a use of drilling parameters may be found in US2019/309614A1.

It has been realized that there is a need to develop improved technology for improving the rock drilling performance.

### SUMMARY OF THE INVENTION

In view of the above, an object of the disclosure is to provide an improved method for real-time adjustment of at least one drilling parameter during rock drilling by a drilling machine which alleviates at least one of the drawbacks of the prior art, or which at least provides a suitable alternative. Another object of the disclosure is to provide an improved control system which at least alleviates at least one of the drawbacks of the prior art, or which at least provides a suitable alternative. Other objects of the disclosure are to provide an improved drilling machine, a computer program, and a computer readable medium, or at least to provide suitable alternatives.

According to a first aspect of the disclosure, at least one of the objects is achieved by a method according to claim 1.

Hence, there is provided a method for real-time adjustment of at least one drilling parameter during rock drilling by a drilling machine. The drilling machine is configured to be operated with an adjustable set of drilling parameters which influence the rock drilling performance.

The method comprises:
- during an ongoing rock drilling operation when the drilling machine is operated with one specific set of drilling parameters of the adjustable set of drilling parameters, measuring, by use of at least one sensor, particle size of particles generated by the rock drilling operation, wherein the measured particles are associated with a point in time when the specific set of drilling parameters are used; and
- adjusting in real-time at least one drilling parameter of the specific set of drilling parameters when the measured particle size fulfils a predefined criterion.

A rock drilling operation as used herein may also be denoted a main rock drilling operation when a drilling tool, e.g. a drill bit, is drilling in rock, in particular in homogenous rock. A collaring drilling operation may be performed before the actual rock drilling operation has begun. Accordingly, the rock drilling operation as disclosed herein is not a collaring drilling operation.

By the provision of a method as disclosed herein, the at least one drilling parameter will be adjusted in real-time when the measured particle size fulfils the predefined criterion. Thereby, the rock drilling operation can be improved while drilling. Consequently, the rock drilling performance can be improved during the rock drilling. The present disclosure is based on a realization that it is advantageous to measure the size of the particles which are generated by the rock drilling operation and use this information for adjusting the one or more drilling parameters during the rock drilling. More particularly, it has been realized that it is advantageous to measure the size of the particles which are associated with the point in time when the specific set of drilling parameters are used. Thereby, the measured particle size will be used as an indicator of the rock drilling performance which is associated with the use of the specific set of drilling parameters. Accordingly, the predefined criterion is used to indicate when the rock drilling performance is not satisfying and that there is a need to adjust at least one of the drilling parameters.

The particles generated by the rock drilling operation may also be denoted cuttings as mentioned in the above, or at least form part of the cuttings. The particles may also be denoted rock particles, i.e. rock particles removed from the rock by the rock drilling operation.

Measuring of particle size may comprise obtaining an average particle size value of the measured particles and using the average particle size value for the comparison with the predefined criterion. Additionally, or alternatively, measuring of particle size may comprise obtaining a particle size distribution of the measured particles and using the particle size distribution value(s) for the comparison with the predefined criterion or in an optimization algorithm, such as in a machine learning algorithm. By way of example, a peak value of the particle size distribution, i.e. a maximum value, may be used for the comparison with the predefined criterion.

Optionally, the steps of measuring particle size and adjusting in real-time at least one drilling parameter are performed continuously in a control loop during the rock drilling operation. Thereby it can be assured that the rock drilling performance is optimized during the complete rock drilling operation, i.e. until the borehole is drilled to its final length.

Optionally, adjusting in real-time the at least one drilling parameter comprises increasing or decreasing the at least one drilling parameter in dependence on the measured particle size, thereby obtaining an updated set of drilling parameters to be used for the ongoing rock drilling operation.

Optionally, the method further comprises:
- evaluating the rock drilling performance as a consequence of the updated set of drilling parameters;
- by use of the evaluation, obtaining a correlation between the updated set of drilling parameters, the rock drilling performance and the measured particle size; and
- using said correlation for determining a need to update a level of adjustment in dependence on the measured particle size.

Accordingly, it has further been realized that it can be advantageous to evaluate the rock drilling performance as a consequence of the updated set of drilling parameters to thereby assess if there is a need to update a level of adjustment in dependence on the measured particle size. In other words, by learning over time, such as by using a machine learning algorithm, the adjustment of the at least one drilling parameter can be improved over time, thereby resulting in a further improved rock drilling performance.

By way of example, the evaluation may be performed by use of one or more sensors which are adapted to measure a value indicative of the rock drilling performance, such as a value indicative of the quality of the borehole and/or a value indicative of how fast the borehole is made, i.e. a drilling speed. The quality of the borehole may relate to a borehole straightness. The evaluation may further include a value relating to a cost for making the borehole, such as a value relating to degradation of rock drilling elements, such as a degradation of a drill bit used for the rock drilling. Additionally, or alternatively, the evaluation may also comprise manual input from a user during and/or after the rock drilling operation.

Optionally, the adjustable set of drilling parameters comprises at least one of the following:
- a rotational speed of a drilling tool of the drilling machine,
- a static load exerted on a drilling tool of the drilling machine,
- a percussion force exerted on a drilling tool of the drilling machine,
- a percussion frequency,
- a feed rate of a drilling tool of the drilling machine,
- a parameter indicative of a particle removal rate of particles generated by the rock drilling operation,
- a flushing parameter, such as flushing pressure and/or flushing speed,
- a torque exerted on a drilling tool of the drilling machine.

Optionally, measuring the particle size of particles is performed by at least one of the following particle size measurement techniques:
- laser diffraction,
- dynamic light scattering,
- electrostatic separation,
- sieve analysis,
- sedimentation,
- electrozone.

Further measurement techniques may be used which are known to the skilled person. However, the above-mentioned measurement techniques have shown to efficiently measure particle size of particles generated by the rock drilling operation.

Optionally, measuring the particle size of particles generated by the rock drilling operation comprises using a time delay parameter indicative of a difference between the point in time when the specific set of drilling parameters are used and when the measurement of particle size is performed, wherein the time delay parameter is used for associating the measured particles with the point in time when the specific set of drilling parameters are used. It has been further realized that it may be advantageous to perform the particle size measurement at a location which is remote from the location where the actual contact between the drilling tool and the rock occurs. More specifically, the equipment used for the measurement will thereby not need to be adapted for the harsh environment where the actual contact between the drilling tool and the rock occurs. As such, by using the time delay parameter, a more correct value of the particle size which is associated with the point in time when the specific set of drilling parameters are used can be obtained. Thereby, the adjustment of the at least one drilling parameter can be improved, resulting in improved rock drilling performance. Preferably, the measurement of particle size is performed outside the borehole, e.g. the measurement may be performed on particles which have been removed from the borehole by a flushing operation.

Optionally, the time delay parameter is variable, such as variable in dependence on a current drilling depth during the ongoing drilling operation. Thereby, a more correct value of the particle size which is associated with the point in time when the specific set of drilling parameters are used can be obtained.

Optionally, the predefined criterion is associated with a type of rock in which the ongoing drilling operation is performed. The particle size of particles generated by a rock drilling operation may vary between different types of rock. Accordingly, by associating the predefined criterion with the type of rock, the rock drilling performance can be further improved.

Optionally, the predefined criterion is associated with the specific set of drilling parameters. Accordingly, by way of example, the predefined criterion may vary in dependence on the specific set of drilling parameters used. For example, for one specific set of drilling parameters it may be expected that the particles should have a size which is in one specific range, whereas for another specific set of drilling parameters it may be expected that the particles should have a size which is in another specific range. Thereby, by associating the predefined criterion with the specific set of drilling parameters, the adjustment of the at least one drilling parameter can be improved. Consequently, the rock drilling performance can thereby be further improved.

Optionally, the method further comprises using the adjusted set of drilling parameters for a subsequent rock drilling operation for drilling another borehole. Thereby, the adjusted set of drilling parameters can be used already at the initiation of the subsequent rock drilling operation. Still optionally, the method comprises using the adjusted set of drilling parameters for a subsequent rock drilling operation for drilling another borehole when the other borehole fulfils a predefined borehole criterion. For example, the predefined borehole criterion may be a predefined maximum distance from where the first rock drilling operation with the adjusted set of drilling parameters were used. Additionally, or alternatively, the predefined borehole criterion may be indicative of that the other borehole is made in similar, or the same, type of rock.

Optionally, the method further comprises keeping the specific set of drilling parameters when the measured particle size is outside of the predefined criterion. Thereby, the rock drilling operation can be continued with the specific set of drilling parameters, i.e. the currently used drilling parameters provides a satisfying rock drilling performance.

Optionally, the method may be used with other sources of information indicative of the current rock drilling performance. For example, a sensor which measures a stress wave shape generated by the rock drilling operation may also be used for evaluating the current rock drilling performance. The at least one drilling parameter may be adjusted when the measured stress wave shape indicates a need to update the at least one drilling parameter. This in combination with the particle size measurement has shown to result in a further improved adjustment of the at least one drilling parameter.

According to a second aspect of the disclosure, at least one of the objects is achieved by a control system according to claim 12.

Hence, there is provided a control system for real-time adjustment of at least one drilling parameter during rock drilling by a drilling machine, wherein the drilling machine is configured to be operated with an adjustable set of drilling parameters which influence the rock drilling performance. The control system comprises at least one sensor for measuring particle size of particles generated by a rock drilling operation of the drilling machine. Furthermore, the control system is configured to perform the method according to any one of the embodiments of the first aspect of the disclosure.

Advantages and effects of the second aspect of the disclosure are analogous to the advantages and effects of the first aspect of the disclosure. It shall also be noted that all embodiments of the first aspect of the disclosure are combinable with all embodiments of the second aspect of the disclosure, and vice versa.

According to a third aspect of the disclosure, at least one of the objects is achieved by a drilling machine according to claim 13.

Hence, there is provided a drilling machine which comprises the control system according to any one of the embodiments of the second aspect of the disclosure.

According to a fourth aspect of the disclosure, at least one of the objects is achieved by a computer program according to claim 14. Hence, there is provided a computer program comprising instructions to cause the control system of the second aspect of the disclosure to execute the method according to any one of the embodiments of the first aspect of the disclosure.

According to a fifth aspect of the disclosure, at least one of the objects is achieved by a computer readable medium according to claim 15. Hence, there is provided a computer readable medium having stored thereon the computer program according to the fourth aspect of the disclosure.

### BRIEF DESCRIPTION OF THE DRAWINGS

With reference to the appended drawings, below follows a more detailed description of embodiments of the disclosure cited as examples.

In the drawings:
Fig. 1 shows a side view of a drilling rig and a drilling machine according to an example embodiment of the present disclosure,
Fig. 2 shows a flowchart of a method according to an example embodiment of the present disclosure; and
Fig. 3 shows a control system according to an example embodiment of the present disclosure.

### DETAILED DESCRIPTION OF EXAMPLE EMBODIMENTS OF THE DISCLOSURE

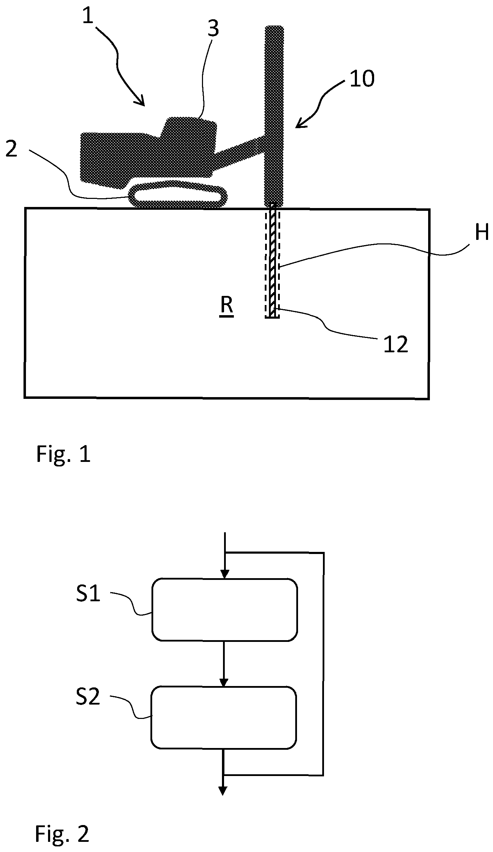

Fig. 1 depicts a drilling rig 1 according to an example embodiment. The drilling rig 1 is herein a movable drilling rig comprising crawler wheels 2 and a user cabin 3. It shall however be understood that the drilling rig may be configured differently. For example, the drilling rig may not comprise a user cabin. Instead of crawler wheels, ordinary wheels may be used etc. In addition, the drilling rig may not have any wheels.

Accordingly, the present disclosure is applicable for any type drilling rig which is adapted to drill in rock.

The drilling rig 1 may be operated by an on-board user, or operator. Additionally, or alternatively, it may be operated remotely and/or it may be autonomously operated, i.e. without any direct human involvement.

The drilling rig 1 comprises a drilling machine 10 according to an example embodiment of the present disclosure. The drilling machine 10 comprises a drilling string with a drilling tool 12, or drill bit, for drilling a borehole H in rock R. The drilling machine may not necessarily be connected to a drilling rig 1, but may in alternative embodiments be a portable device, such as a hand-held device. The drilling machine 10 may be adapted to perform rock drilling by any one of percussion drilling, rotary drilling and percussion assisted rotary drilling.

In the shown embodiment, the drilling machine 10 is adapted to perform a rock drilling operation by rotating the drilling tool 12 at a specific rotational speed while also feeding the drilling tool into the borehole H by application of a static load exerted on the drilling tool 12. In addition, a percussion force with a specific percussion frequency is exerted on the drilling tool 12 during the rock drilling operation.

The drilling machine 10 may for example be adapted to drill boreholes with a diameter in the range of 20-450 mm. However, the present disclosure is also applicable for drilling machines adapted to drill boreholes with smaller or larger diameters. The drilling machine 10 may also be adapted to drill boreholes with varying depths, such as in a range of 0.1 m to 500 m.

During the rock drilling operation, particles are generated. The particles may be removed by a flushing operation, such as by use of pressurized air which is injected into the borehole H.

With reference to fig. 2, an example embodiment of a method according to the present disclosure is shown. The method can be performed by the drilling machine 10 as shown in fig. 1. Accordingly, the drilling machine 10 may further comprise at least one sensor (not shown) for measuring particle size of the particles generated by the rock drilling operation. The at least one sensor for measuring particle size may be located outside the borehole H. Additionally, or alternatively, at least one sensor for measuring particle size may be located inside the borehole H, such as at a position adjacent to, on or in the drilling tool 12, or drill bit.

The method performs real-time adjustment of at least one drilling parameter during rock drilling by the drilling machine 10.

The drilling machine 10 is configured to be operated with an adjustable set of drilling parameters which influence the rock drilling performance.

For example, the adjustable set of drilling parameters may comprise at least one of the following:
- a rotational speed of the drilling tool 12 of the drilling machine 10,
- a static load exerted on the drilling tool 12 of the drilling machine 10,
- a percussion force exerted on the drilling tool 12 of the drilling machine 10,
- a percussion frequency,
- a feed rate of the drilling tool 12 of the drilling machine 10,
- a parameter indicative of a particle removal rate of the particles generated by the rock drilling operation,
- a flushing parameter, such as flushing pressure and/or flushing speed,
- a torque exerted on the drilling tool 12 of the drilling machine 10.

The method comprises:
S1: during an ongoing rock drilling operation when the drilling machine 10 is operated with one specific set of drilling parameters of the adjustable set of drilling parameters, measuring, by use of the at least one sensor, particle size of particles generated by the rock drilling operation. The measured particles are associated with a point in time when the specific set of drilling parameters are used.

The method further comprises:
S2: adjusting in real-time at least one drilling parameter of the specific set of drilling parameters when the measured particle size fulfils a predefined criterion.

By way of example, the power P exerted on the rock R at the contact between the drilling tool 12 and the rock R may be defined as P = E * f, where E is the percussion force, or percussion energy, exerted on the drilling tool 12 of the drilling machine 10, and f is the percussion frequency. Accordingly, the parameters E and f may be adjusted in real-time when the measured particle size fulfils the predefined criterion. Thereby, the rock drilling performance can be improved during the rock drilling operation. For example, by adjusting the at least one rock drilling parameter in real-time, the borehole quality may be improved, such as the straightness of the borehole H. Additionally, or alternatively, by adjusting the at least one rock drilling parameter in real-time, the time for making the borehole H may be reduced.

Any one of the above-mentioned rock drilling parameters may be adjusted in real-time during the ongoing rock drilling operation.

For example, the predefined criterion may be associated with particles with a particle size which is smaller than a predefined threshold value. Too small particles may be indicative of a too low percussion force or a too high percussion force. It may also be indicative of too many percussions in the same area, implying crushing of particles several times, e.g. too slow or too high rotation. It may also be indicative of a too slow particle removal rate, i.e. the flushing is performed at a too slow flushing rate. In another example, the predefined criterion may be associated with a particle size distribution. How the particles are distributed may be used in a control loop to set the drilling parameter(s) so that the desired particle size distribution is achieved.

The steps of measuring particle size and adjusting in real-time at least one drilling parameter, S1 and S2, are preferably performed continuously in a control loop during the rock drilling operation. This is indicated in fig. 2 by the arrow going from the box S2 back to the box S1.

The adjusting in real-time of the at least one drilling parameter may comprise increasing or decreasing the at least one drilling parameter in dependence on the measured particle size, thereby obtaining an updated set of drilling parameters to be used for the ongoing rock drilling operation. For example, the real-time adjustment may comprise to increase or decrease the rotational speed of the drilling tool 12.

The method may further comprise:
- evaluating the rock drilling performance as a consequence of the updated set of drilling parameters;
- by use of the evaluation, obtaining a correlation between the updated set of drilling parameters, the rock drilling performance and the measured particle size; and
- using said correlation for determining a need to update a level of adjustment in dependence on the measured particle size. Accordingly, the level of adjustment may be updated when it is determined that there is a need to update the level of adjustment. Additionally, or alternatively, the correlation may be used for updating the predefined criterion.

For example, the evaluation of the rock drilling performance may be an evaluation of how straight the borehole H is. Additionally, or alternatively, the evaluation may be an evaluation of how fast the borehole H is made. Additionally, or alternatively, the evaluation may be an evaluation of how fast the borehole H is made in relation to degradation of the drilling machine 10, such as degradation of the drilling tool 12.

By way of example, the updating of the level of adjustment and/or the predefined criterion may be performed by use of a machine learning algorithm. Thereby, the rock drilling performance can be continuously improved. The predefined criterion and/or the level of adjustment of the at least one drilling parameter may initially be manually set by a user. The user may set the predefined criterion and/or the level of adjustment of the at least one drilling parameter according to the user's current knowledge about a satisfying rock drilling performance. Thereafter, the predefined criterion and/or the level of adjustment may be continuously updated based on the evaluation of the rock drilling performance. Accordingly, the method will be further improved when more information is obtained about the rock drilling performance and its correlation to the updated set of drilling parameters and the measured particle size.

The measuring of the particle size of particles may be performed by at least one of the following particle size measurement techniques:
- laser diffraction,
- dynamic light scattering,
- electrostatic separation,
- Sieve analysis,
- sedimentation,
- electrozone.

Other particle size measurement techniques may be optical, ultrasound or electric field methods and also mechanical methods such as centrifugal methods.

Any other known technology for measuring particle size may be used.

Furthermore, measuring the particle size of particles generated by the rock drilling operation may comprise using a time delay parameter Δt which is indicative of a difference between the point in time when the specific set of drilling parameters are used and when the measurement of particle size is performed. The time delay parameter Δt is used for associating the measured particles with the point in time when the specific set of drilling parameters are used.

For example, the particles generated from the borehole H in fig. 1 may be measured when they have been removed from the borehole H, e.g. at a position close to the drilling machine 10 and the drilling rig 1. As such, even though at least one drilling parameter already may have been adjusted during a time window defined by the time delay parameter Δt, the measured particle size can be associated with the specific set of drilling parameters that was used when the particles were generated. Thereby, it can be assured that the adjustment of the at least one drilling parameter will be based on the size of particles which were generated at the time when the specific set of drilling parameters was used.

The time delay parameter Δt may be variable. Preferably, the time delay parameter Δt is variable in dependence on a current drilling depth during the ongoing drilling operation.

Furthermore, the predefined criterion may be associated with a type of rock in which the ongoing drilling operation is performed. More particularly, different particle sizes can be expected when drilling in different types of rock. As such, varying the predefined criterion based on the type of rock may result in further improved rock drilling performance.

In addition, the predefined criterion may be associated with the specific set of drilling parameters. Accordingly, the predefined criterion may also be varied based on the specific set of drilling parameters. This may result in a database comprising different predefined criterions which are associated with e.g. different specific sets of drilling parameters, different types of rock etc. The database may be continuously updated, e.g. by use of the above-mentioned machine learning algorithm. The database may be shared between different drilling rigs and drilling machines. The database may additionally, or alternatively, be fed by data from different drilling rigs and drilling machines.

The method may further comprise using the adjusted set of drilling parameters for a subsequent rock drilling operation for drilling another borehole. Thereby, when drilling the other borehole, the rock drilling performance can already at initiation of the rock drilling be at a higher level than when drilling the first borehole H. For example, the method may comprise using the adjusted set of drilling parameters for a subsequent rock drilling operation for drilling another borehole when the other borehole fulfils a predefined borehole criterion. For example, the predefined borehole criterion may be a predefined maximum distance from where the first rock drilling operation with the adjusted set of drilling parameters was used. Additionally, or alternatively, the predefined borehole criterion may be indicative of that the other borehole is made in similar, or the same, type of rock. Additionally, or alternatively, the predefined borehole criterion may be indicative of a time period from when the previous borehole H was drilled.

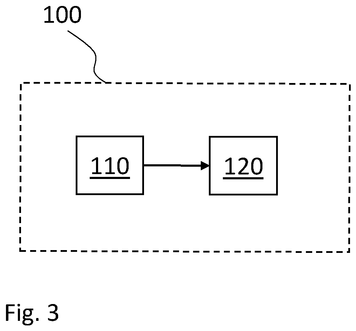

With reference to fig. 3, a schematic illustration of a control system 100 according to the present disclosure is depicted. The control system 100 is used for real-time adjustment of at least one drilling parameter during rock drilling by a drilling machine 10. The drilling machine 10 is configured to be operated with an adjustable set of drilling parameters which influence the rock drilling performance. The control system 100 comprises at least one sensor 110 for measuring particle size of particles generated by a rock drilling operation of the drilling machine. The control system 100 is further configured to perform the method according to any one of the embodiments of the first aspect of the disclosure. For example, the control system 100 may comprise one or more control units 120 for performing the method. As illustrated in fig. 3 by an arrow, the at least one sensor 110 may communicate with the control unit 120, i.e. it may continuously or intermittently transmit values of the measured particle size to the control unit 120. The sensor 110 and the control unit 120 may be provided on the drilling machine 10 and/or on the drilling rig 1. However, other configurations are also possible. For example, the control unit 120 may be an off-board control unit, such as provided at a remote location from the drilling rig 1 and the drilling machine 10. Accordingly, the communication, which may be a one-way or two-way communication, between the sensor 110 and the control unit 120 may be a wired and/or wireless communication.

The control unit 120 is herein an electronic control unit. It may comprise processing circuitry which is adapted to run a computer program as disclosed herein. The control unit 120 may comprise hardware and/or software for performing the method according to the invention. In an embodiment, the control unit 120 may be denoted a computer. The control unit 120 may be constituted by one or more separate sub-control units.

It is to be understood that the present disclosure is not limited to the embodiments described above and illustrated in the drawings; rather, the skilled person will recognize that many changes and modifications may be made within the scope of the appended claims.

## Claims

1. A method for real-time adjustment of at least one drilling parameter during rock drilling by a drilling machine (10), wherein the drilling machine (10) is configured to be operated with an adjustable set of drilling parameters which influence the rock drilling performance, the method comprising:
- during an ongoing rock drilling operation when the drilling machine (10) is operated with one specific set of drilling parameters of the adjustable set of drilling parameters, measuring (S1), by use of at least one sensor, particle size of particles generated by the rock drilling operation, wherein the measured particles are associated with a point in time when the specific set of drilling parameters are used; and
- adjusting (S2) in real-time at least one drilling parameter of the specific set of drilling parameters when the measured particle size fulfils a predefined criterion;
wherein adjusting in real-time the at least one drilling parameter comprises increasing or decreasing the at least one drilling parameter in dependence on the measured particle size, thereby obtaining an updated set of drilling parameters to be used for the ongoing rock drilling operation;
**characterised in that** the method further comprising:
- evaluating the rock drilling performance as a consequence of the updated set of drilling parameters;
- by use of the evaluation, obtaining a correlation between the updated set of drilling parameters, the rock drilling performance and the measured particle size; and
- using said correlation for determining a need to update a level of adjustment in dependence on the measured particle size.

2. The method according to claim 1, wherein the steps of measuring particle size and adjusting in real-time at least one drilling parameter are performed continuously in a control loop during the rock drilling operation.

3. The method according to any one of the preceding claims, wherein the adjustable set of drilling parameters comprises at least one of the following:
- a rotational speed of a drilling tool (12) of the drilling machine (10),
- a static load exerted on a drilling tool (12) of the drilling machine (10),
- a percussion force exerted on a drilling tool (12) of the drilling machine (10),
- a percussion frequency,
- a feed rate of a drilling tool (12) of the drilling machine (10),
- a parameter indicative of a particle removal rate of particles generated by the rock drilling operation,
- a flushing parameter, such as flushing pressure and/or flushing speed,
- a torque exerted on a drilling tool (12) of the drilling machine (10).

4. The method according to any one of the preceding claims, wherein measuring the particle size of particles is performed by at least one of the following particle size measurement techniques:
- laser diffraction,
- dynamic light scattering,
- electrostatic separation,
- sieve analysis,
- sedimentation,
- electrozone.

5. The method according to any one of the preceding claims, wherein measuring the particle size of particles generated by the rock drilling operation comprises using a time delay parameter indicative of a difference between the point in time when the specific set of drilling parameters are used and when the measurement of particle size is performed, wherein the time delay parameter is used for associating the measured particles with the point in time when the specific set of drilling parameters are used.

6. The method according to claim 5, wherein the time delay parameter is variable, such as variable in dependence on a current drilling depth during the ongoing drilling operation.

7. The method according to any one of the preceding claims, wherein the predefined criterion is associated with a type of rock in which the ongoing drilling operation is performed.

8. The method according to any one of the preceding claims, wherein the predefined criterion is associated with the specific set of drilling parameters.

9. The method according to any one of the preceding claims, further comprising using the adjusted set of drilling parameters for a subsequent rock drilling operation for drilling another borehole.

10. A control system (100) for real-time adjustment of at least one drilling parameter during rock drilling by a drilling machine (10), wherein the drilling machine (10) is configured to be operated with an adjustable set of drilling parameters which influence the rock drilling performance, the control system (100) comprising at least one sensor (110) for measuring particle size of particles generated by a rock drilling operation of the drilling machine, wherein the control system (100) is configured to perform the method according to any one of claims 1-9.

11. A drilling machine (10) comprising the control system (100) according to claim 10.

12. A computer program comprising instructions to cause the control system (100) of claim 10 to execute the method according to any one of claims 1-9.

13. A computer readable medium having stored thereon the computer program according to claim 12.

## Patentansprüche

1. Verfahren zur Echtzeiteinstellung von mindestens einem Bohrparameter während des Felsbohrens durch eine Bohrmaschine (10), wobei die Bohrmaschine (10) so konfiguriert ist, dass sie mit einem einstellbaren Satz von Bohrparametern betrieben wird, die die Felsbohrleistung beeinflussen, wobei das Verfahren Folgendes umfasst:
- während eines laufenden Felsbohrvorgangs, wenn die Bohrmaschine (10) mit einem bestimmten Satz von Bohrparametern aus dem einstellbaren Satz von Bohrparametern betrieben wird, Messen (S1), unter Verwendung von mindestens einem Sensor, der Partikelgröße von Partikeln, die durch den Felsbohrvorgang erzeugt werden, wobei die gemessenen Partikel einem Zeitpunkt zugeordnet werden, zu dem der bestimmte Satz von Bohrparametern verwendet wird; und
- Einstellen (S2), in Echtzeit, mindestens eines Bohrparameters des spezifischen Satzes von Bohrparametern, wenn die gemessene Partikelgröße ein vordefiniertes Kriterium erfüllt;
wobei das Anpassen des mindestens einen Bohrparameters in Echtzeit das Erhöhen oder Verringern des mindestens einen Bohrparameters in Abhängigkeit von der gemessenen Partikelgröße umfasst, wodurch ein aktualisierter Satz von Bohrparametern erhalten wird, die für den laufenden Felsbohrvorgang zu verwenden sind;
**dadurch gekennzeichnet, dass** das Verfahren weiter Folgendes umfasst:
- Auswerten der Felsbohrleistung als Folge der aktualisierten Bohrparameter;
- durch Verwenden der Auswertung, Erhalten einer Korrelation zwischen dem aktualisierten Satz von Bohrparametern, der Felsbohrleistung und der gemessenen Partikelgröße; und
- Verwenden der Korrelation zum Bestimmen der Notwendigkeit einer Aktualisierung des Einstellungsgrades in Abhängigkeit von der gemessenen Partikelgröße.

2. Verfahren nach Anspruch 1, wobei die Schritte des Messens der Partikelgröße und des Einstellens mindestens eines Bohrparameters in Echtzeit während des Felsbohrvorgangs kontinuierlich in einem Regelkreis durchgeführt werden.

3. Verfahren nach einem der vorstehenden Ansprüche, wobei der einstellbare Satz von Bohrparametern mindestens eines des Folgenden umfasst:
- eine Drehzahl eines Bohrwerkzeugs (12) der Bohrmaschine (10),
- eine statische Last, die auf ein Bohrwerkzeug (12) der Bohrmaschine (10) ausgeübt wird,
- eine Schlagkraft, die auf ein Bohrwerkzeug (12) der Bohrmaschine (10) ausgeübt wird,
- eine Schlagfrequenz,
- eine Vorschubgeschwindigkeit eines Bohrwerkzeugs (12) der Bohrmaschine (10),
- einen Parameter, der die Partikelentfernungsrate von durch den Felsbohrvorgang erzeugten Partikel angibt,
- einen Spülparameter, wie beispielsweise Spüldruck und/oder Spülgeschwindigkeit,
- ein Drehmoment, das auf ein Bohrwerkzeug (12) der Bohrmaschine (10) ausgeübt wird.

4. Verfahren nach einem der vorstehenden Ansprüche, wobei die Messung der Partikelgröße von Partikeln durch mindestens eines der folgenden Partikelgrößenmessverfahren durchgeführt wird:
- Laserbeugung,
- dynamische Lichtstreuung,
- elektrostatische Trennung,
- Siebanalyse,
- Sedimentation,
- Elektrozone.

5. Verfahren nach einem der vorstehenden Ansprüche, wobei das Messen der Partikelgröße von Partikeln, die durch den Felsbohrvorgang erzeugt werden, das Verwenden eines Zeitverzögerungsparameters umfasst, der eine Differenz zwischen dem Zeitpunkt, zu dem der spezifische Satz von Bohrparametern verwendet wird, und dem Zeitpunkt, zu dem die Messung der Partikelgröße durchgeführt wird, angibt, wobei der Zeitverzögerungsparameter verwendet wird, um die gemessenen Partikel dem zuzuordnen, zu dem der spezifische Satz von Bohrparametern verwendet wird.

6. Verfahren nach Anspruch 5,
wobei der Zeitverzögerungsparameter variabel ist, beispielsweise in Abhängigkeit von einer aktuellen Bohrtiefe während des laufenden Bohrvorgangs.

7. Verfahren nach einem der vorstehenden Ansprüche, wobei das vordefinierte Kriterium einer Art von Gestein zugeordnet ist, in dem der laufende Bohrvorgang durchgeführt wird.

8. Verfahren nach einem der vorstehenden Ansprüche, wobei das vordefinierte Kriterium dem spezifischen Satz von Bohrparametern zugeordnet ist.

9. Verfahren nach einem der vorstehenden Ansprüche, weiter umfassend das Verwenden des eingestellten Satzes von Bohrparametern für einen nachfolgenden Felsbohrvorgang zum Bohren eines weiteren Bohrlochs.

10. Steuerungssystem (100) zur Echtzeiteinstellung von mindestens einem Bohrparameter während des Felsbohrens durch eine Bohrmaschine (10), wobei die Bohrmaschine (10) so konfiguriert ist, dass sie mit einem einstellbaren Satz von Bohrparametern betrieben wird, die die Felsbohrleistung beeinflussen, wobei das Steuerungssystem (100) mindestens einen Sensor (110) zum Messen der Partikelgröße von Partikeln umfasst, die durch einen Felsbohrvorgang der Bohrmaschine erzeugt werden, wobei das Steuerungssystem (100) so konfiguriert ist, dass es das Verfahren nach einem der Ansprüche 1 bis 9 ausführt.

11. Bohrmaschine (10) umfassend ein Steuerungssystem (100) nach Anspruch 10.

12. Computerprogramm, das Anweisungen umfasst, um das Steuerungssystem (100) nach Anspruch 10 zu veranlassen, das Verfahren nach einem der Ansprüche 1 bis 9 auszuführen.

13. Computerlesbares Medium, auf dem das Computerprogramm nach Anspruch 12 gespeichert ist.

## Revendications

1. Procédé de réglage en temps réel d'au moins un paramètre de forage pendant un forage de roche par une machine de forage (10), dans lequel la machine de forage (10) est configurée pour être exploitée avec un ensemble réglable de paramètres de forage qui influence les performances de forage de roche, le procédé comprenant :
- pendant une opération de forage de roche en cours lorsque la machine de forage (10) est exploitée avec un ensemble spécifique de paramètres de forage de l'ensemble réglable de paramètres de forage, la mesure (S1), en utilisant au moins un capteur, d'une taille de particule de particules générées par l'opération de forage de roche, dans lequel les particules mesurées sont associées à un moment où l'ensemble spécifique de paramètres de forage est utilisé ; et
- le réglage (S2) en temps réel d'au moins un paramètre de forage de l'ensemble spécifique de paramètres de forage lorsque la taille de particule mesurée respecte un critère prédéfini ; dans lequel le réglage en temps réel du au moins un paramètre de forage comprend l'augmentation ou la diminution du au moins un paramètre de forage en fonction de la taille de particule mesurée, obtenant ainsi un ensemble actualisé de paramètres de forage à utiliser pour l'opération de forage de roche en cours ;
**caractérisé en ce que** le procédé comprenant en outre :
- l'évaluation des performances de forage de roche découlant de l'ensemble actualisé de paramètres de forage ;
- en utilisant l'évaluation, l'obtention d'une corrélation entre l'ensemble actualisé de paramètres de forage, les performances de forage de roche et la taille de particule mesurée ; et
- l'utilisation de ladite corrélation pour déterminer un besoin d'actualiser un niveau de réglage en fonction de la taille de particule mesurée.

2. Procédé selon la revendication 1, dans lequel les étapes de mesure de la taille de particule et de réglage en temps réel d'au moins un paramètre de forage sont réalisées en continu dans une boucle de commande pendant l'opération de forage de roche.

3. Procédé selon l'une quelconque des revendications précédentes, dans lequel l'ensemble réglable de paramètres de forage comprend au moins un des éléments suivants :
- une vitesse de rotation d'un outil de forage (12) de la machine de forage (10),
- une charge statique exercée sur un outil de forage (12) de la machine de forage (10),
- une force de percussion exercée sur un outil de forage (12) de la machine de forage (10),
- une fréquence de percussion,
- une vitesse d'avance d'un outil de forage (12) de la machine de forage (10),
- un paramètre indicatif d'un taux d'élimination de particule de particules générées par l'opération de forage de roche,
- un paramètre de rinçage, tel que pression de rinçage et/ou vitesse de rinçage,
- un couple exercé sur un outil de forage (12) de la machine de forage (10).

4. Procédé selon l'une quelconque des revendications précédentes, dans lequel la mesure de la taille de particule de particules est réalisée par au moins une des techniques de mesure de taille de particule suivantes :
- diffraction laser,
- diffusion dynamique de la lumière,
- séparation électrostatique,
- analyse granulométrique,
- sédimentation,
- électrozone.

5. Procédé selon l'une quelconque des revendications précédentes, dans lequel la mesure de la taille de particule de particules générées par l'opération de forage de roche comprend l'utilisation d'un paramètre de retard temporel indicatif d'une différence entre le moment où l'ensemble spécifique de paramètres de forage est utilisé et le moment où la mesure de la taille de particule est réalisée,
dans lequel le paramètre de retard temporel est utilisé pour associer les particules mesurées et le moment où l'ensemble spécifique de paramètres de forage est utilisé.

6. Procédé selon la revendication 5,
dans lequel le paramètre de retard temporel est variable, tel que variable en fonction de la profondeur réelle de forage pendant l'opération de forage en cours.

7. Procédé selon l'une quelconque des revendications précédentes, dans lequel le critère prédéfini est associé à un type de roche dans lequel l'opération de forage en cours est réalisée.

8. Procédé selon l'une quelconque des revendications précédentes, dans lequel le critère prédéfini est associé à l'ensemble spécifique de paramètres de forage.

9. Procédé selon l'une quelconque des revendications précédentes, comprenant en outre l'utilisation de l'ensemble réglé de paramètres de forage pour une opération de forage de roche ultérieure pour le forage d'un autre trou de forage.

10. Système de commande (100) de réglage en temps réel d'au moins un paramètre de forage pendant un forage de roche par une machine de forage (10), dans lequel la machine de forage (10) est configurée pour être exploitée avec un ensemble réglable de paramètres de forage qui influence les performances de forage de roche,
le système de commande (100) comprenant au moins un capteur (110) de mesure de la taille de particule de particules générées par une opération de forage de roche de la machine de forage, dans lequel le système de commande (100) est configuré pour réaliser le procédé selon l'une quelconque des revendications 1 à 9.

11. Machine de forage (10) comprenant le système de commande (100) selon la revendication 10.

12. Programme informatique comprenant des instructions pour amener le système de commande (100) selon la revendication 10 à exécuter le procédé selon l'une quelconque des revendications 1 à 9.

13. Support lisible par ordinateur sur lequel est stocké le programme informatique selon la revendication 12.
